# EUROPEAN PATENT APPLICATION

(11) **EP 3 735 828 A1**
(43) Date of publication of application: **11.11.2020**
(21) Application number: 20175577.4
(22) Date of filing: 03.12.2015
(51) Int. Cl.: A01N 59/06, A01N 25/02, A01N 25/08, A62D 101/04, A01P 1/00, A01P 3/00, C01F 5/00

(54) **IMPROVED PATHOGEN INHIBITOR**

(30) Priority: 14.01.2015 AU 2015900093; 05.11.2015 AU 2015904534
(62) Divisional of application: 15877383.8
(71) Applicant: Calix Limited, Pymble, NSW 2073 (AU)
(72) Inventor: Sceats, Mark, Pymble, New South Wales 2073 (AU); Hodgson, Philip, Pymble, New South Wales 2073 (AU)
(74) Representative: Fleuchaus, Michael A.

(57) **Abstract**

A pathogen inhibitor concentrate for use in an ecosystem. The concentrate comprises particles having a uniform distribution of magnesium hydroxide and magnesium peroxide with a relative molar ratio to control the bioactivity of the concentrate by providing sufficient alkali and reactive oxygen species to meet the requirements of the ecosystem, wherein the requirements of the ecosystem are defined as maintaining an aerobic system, killing pathogenic, anaerobic microbes or producing a sterile ecosystem, and the requirements of the ecosystem are determined based on the redox potential and pH of the ecosystem. At least one of the magnesium hydroxide and the magnesium peroxide make up at least 30% by weight of the concentrate.

## Description

### TECHNICAL FIELD

The present invention relates broadly to a formulation for bio-active materials, where the bioactivity applies to a broad spectrum of viruses, bacteria, fungi as a pathogen inhibitor depending on the target, host and dose. Applications in the fields of agriculture, aquaculture and health products are examples described for the application of the formulation.

### BACKGROUND

In agriculture, the ecosystems that are managed by farmers are the soil and the leaf ecosystems. The focus of this invention is the leaf ecosystem. It is understood that the surfaces of leaves are aerobic ecosystems, and plant disease generally occurs when that ecosystem becomes anaerobic, so that pathological microorganisms thrive, and eventually infect and kill the plant. The plant's internal defence includes the generation of Reactive Oxygen Species (ROS), generally in a burst that heralds the onset of infection. ROS species include the superoxide ion, the hydroperoxyl radical, hydrogen peroxide, and the hydroxyl and atomic oxygen radicals. The plant does not generally inject ROS onto the leaf, so this mechanism is reserved for internal defence. The reason is that ROS species attack the cell structure itself, so that sustained high ROS generation is not possible without damage to the cell. ROS is produced by certain gram positive bacteria, such as Lactobacillus, which lives symbiotically on the leaves of the host, and its ROS can help sustain the aerobic conditions on the leaf. However, disease regularly occurs, and the onset of disease if the growth of colonies of microbes on the leaf surface with the concurrent transformation of the surface into an anaerobic environment. That is, the ROS from symbiotic sources, and other defensive mechanisms are overcome. The usual approach to such outbreaks by farmers is to spray the leaves with fungicides and bactericides. However, it is well established that these fungicides are toxic, and the pathogens have a capacity to evolve such that the impact of a fungicide gradually decreases with multiple applications. The leaf has the capacity to absorb fertilisers through the stomata, and this provides a means of direct fertilisation for the production of chlorophyll in the leaf. There is a need for substances that can be applied to the leaf ecosystem which can regenerate the aerobic environment, and remove disease, without being toxic to humans, animals that eat the plant, and to the plant itself, and which provide magnesium as a fertilizer that promotes plant growth.

In aquaculture, the ecosystems that are directly managed by farmers are the water and the bottom of the pen. A local ecosystem is the skin of the host (fish, prawns), which move between these systems. The primary focus of this invention is associated with the need to maintain an aerobic environment in those ecosystems that can be directly managed. These ecosystems are understood to be critical to growth. The aquatic ecosystem also supports a wide variety of organisms apart from the host, some of which are food for the host, and others which live in a symbiotic relationship. Aquaculture is generally an intensive activity, where the population density of the host is higher than natural waters, and the hosts are generally fed with feed that is distributed in the water, and often there is an oversupply of feed. This, plus the waste from the host can accumulate and the ecosystem can become stressed, and disease is readily generated, often through the growth of pathogenic microbes, initially on exposed skin, The characteristics of an unhealthy ecosystem is a low oxygen content, and a low pH often accompanied by a sulphides and other toxic compounds. There is a need for substances that can be applied to the aquatic ecosystem which can regenerate the aerobic environment, and remove disease, without being toxic to humans, the host species animals, and which provide calcium and magnesium as a fertilizer that promotes the host growth.

In health products, external organs (such as the skin), the stomach, and mouth are ecosystems that host a wide range of micro-organisms, and which include pathogens characteristic of diseases or infestations when these ecosystems are disrupted. Of interest in this invention are the aerobic ecosystems on the skin and mouth, for which there are a wide variety of creams and pastes that are used to maintain the health. To an extent, relevant ecosystems include food.

There has been an extensive development of nano-materials with biocide properties, and in particular, of nano-magnesia MgO and nano-zinc oxide ZnO. An example of a biocide is "Antibacterial characteristics of magnesium oxide powder", J. Sawei et al. World Journal of Microbiology and Biotechnology, 16, Issue 2, pp 187-194 (2000) and T. Yin and Y. He, "Antibacterial activities of magnesium oxide nanoparticles against foodborne pathogens" J. Nanopart. Res. 13, 6877-6885.

In the study by Sawai et al, the objective was to make high surface area MgO with particle sizes below about 50 nm. In trials of these materials, the MgO particles rapidly react with water to form nano-magnesium hydroxide Mg(OH)₂. Prior art references to nano-MgO are ascribed herein to nano-Mg(OH)₂. These hydrated nano-materials exhibit broad spectrum bioactivity response to vims, bacteria and fungi. The powder, including the hydrated nano-powder, also has as an ability to deactivate toxic materials such as chemical warfare agents.

In a paper published by T. Yin and Y. Lu, it was demonstrated that nano-MgO particles have a strong biocide activity against two foodborne pathogens, namely Escherichia Coli and Salmonella. This work is important because nano-MgO/Mg(OH)₂ is not believed to be toxic to humans or animals, and has a positive impact on plants through the supply of magnesium as a fertilizer. For example, seven log reductions in E. Coli were observed at a dosage rate of 8 g/litre solids, and dosages of 1 g/litre supressed growth, and that 3 g/litre would kill all cells within 24 hrs. While Mg(OH)2is relatively insoluble, it rapidly dissolves in low pH environments, especially at the pH of digestive systems. This would be true of nano-MgO/Mg(OH)₂ because the dissolution rate is faster the higher the surface area. The ecosystems described above are either mildly acidic or strongly acidic.

US Patent No. 6,827,766 B2 claims a decontamination product comprising nanoparticles including MgO and Mg(OH)₂, selective biocides and a liquid carrier, including water. The biocide properties are significantly enhanced by the presence of the nano-particles. The decontamination processes include a liquid spray, fog, aerosol paste, gel, wipe, vapour or foam. While the claims are limited to the requirement of adding an existing biocide as an adjuvant to the product, the examples disclosed teach that the nano-particles, in the liquid carriers, had an effective, long-term biocide activity without the adjuvant. Specifically, their example 3 shows that 5/1 water/oil emulsion with 2% nano-MgO, CaO, and ZnO solids had such properties, notably without the requirement of a biocide.

The impact of the particle size would seem to be important. US Patent No. 2,576,731 A (Thomson) discloses the use of magnesium hydroxide slurry, made from a standard magnesium oxide, as the basis for a foliar spray as a carrier for active biocides for both insects and fungi where the benefits are associated with the ability of the alkaline particles to absorb active biocides to render them insoluble, and the strong adherence of the particles on the leaves of the plants such that the biocide can act over many washings of the leaf. That patent describes the role of the magnesium hydroxide as having no insecticidal or germicidal activity. In the context of this invention, the important teaching of that patent is the adherence of magnesium hydroxide.

This view was supported by a paper published by Motoike et al. "Antiviral activities of heated dolomite powder" Biocontrol Sci.13(4): 131-8 2008 in which processed dolomite is shown to exhibit anti-viral activity. Patent US 2009/0041818 A1 claims an anti-viral agent which is a mixture of an oxide and a hydroxide, in which it is taught that hydroxide ions are produced by the reaction of the oxide with a hydroxide. It is claimed that many materials can provide the hydroxide, among which is Mg(OH)₂, and the oxide is preferably MgO. The relevant disclosure of this prior art is that the biocide activity of such conventional slurries is primarily transient and thus a manufactured magnesium hydroxide, or hydrated calcined dolomite slurry, does not have a significant long term biocide effect. Without being limited by theory, this work suggests that the bioactive chemical species in such a hydroxide slurry are naturally present, but their concentration is too low for a sustained impact on microbes. The present invention may seek to overcome this limitation.

The most plausible theory of why pathological fungal growth is suppressed by chemical processes in nano-Mg(OH)₂ is that the generation of ROS. ROS have a high redox-potential. There are equilibria between these species in water that is largely regulated by the H, and at the pH near a nano-Mg(OH)₂ grain, around 10.4, the perhydroxyl anion dominates. Plants can ramp up the production of ROS inside their cells as a defence against pathogenic microbial attack, with the ROS attacking the primitive, generally anaerobic cell walls of pathogenic fungus and bacteria that enter the plant cells. In response, fungus can produce chemical species that react and neutralise the ROS. The same model is true of pathogenic bacteria, in particular the anaerobic gram-negative bacteria. The symbiosis is associated with the relationship between the plant ROS and the beneficial gram positive bacteria that live on the leaves, and which are essential to a healthy environment for growth. Gram positive bacteria are generally beneficial and aerobic, and the ROS increases the oxygen level in the environment. For example, as demonstrated in the case of rice blast fungus: Kun Huang, Kirk J. Czymmek, Jeffrey L. Caplan, James A. Sweigard & Nicole M. Donofrio (2011). Further, it has been established that beneficial gram positive bacteria such as Lactobacillus produce, and exude ROS, and such aerobic bacteria often live symbiotically in the leaf ecosystems of plants, and assist the plant in resisting pathogenic, anaerobic microbes.

At the atomic level, it is evident from prior art that the long term biological activity of nano-Mg(OH)₂ slurry is associated with is ability to produce ROS. The active species are not well understood. In general terms, small crystal grains in nanoparticles have, by definition, a high proportion of their crystalline surfaces which are formed at the high energy surfaces, and it is well understood that such surfaces of oxide nanoparticles are the source of energetic oxidants, such as the ROS species. In the case of Mg(OH)2, techniques such as Electron Paramagnetic Resonance has detected all of the radical species described above as ROS on normal crystals, albeit at low concentrations. ROS radicals in solution can recombine, and the bio-activity impact of ROS would degrade by radical recombination. In the presence of Mg(OH)₂, it is believed that the rate of dissipation of ROS can be substantially reduced, if not supressed by the generation of magnesium peroxide MgO₂. Magnesium peroxide is a stable crystalline material, and is usually formed in a mixture with hydrogen peroxide H₂O₂, water and excess MgO. It is stable in this form at ambient temperature (I. I. Vol'nov, and E. I. Latysheva, "Thermal stability of magnesium peroxide" Izvestiya Akademii Nauk SSSR, Seriya Khimicheskaya, No. 1, pp. 13-18, January, 1970). Therefore, nano-Mg(OH)₂ can not only form ROS at the grain boundaries but also the ROS species can be stabilized on the grain surfaces. The ROS species are stored on the nano-grain surfaces, and would be released by the change in the equilibria associated with pathogen attack, and general dissolution of the nano-Mg(OH)₂ to supply magnesium to the plant as a fertilizer.

In summary, a reasonable model for the bioactivity of nano-Mg(OH)₂ is that each particle is a nanoscale crystalline grain that has a relatively high concentration of ROS precursors, such as magnesium peroxide, which is stabilised on the energetic surfaces of the grain, and the bioactivity arises from the enhancement of the plant's own natural defence systems which form ROS to provide the aerobic environment within the cell that suppresses pathogenic microbes that have infected the plant. The application of a substance to the surface of the cell which can produce ROS on the surface complements the internal generation of ROS if infection occurs, and may supplement the ROS produced, for example, by gram positive aerobic bacteria such as lactobacillus. This effect is enhanced by the pH of the Mg(OH)₂, at 10.4 which may neutralise acids extruded by pathogens; the net positive particle charge from hydrolysis which attracts the particles to negatively charged surfaces of certain microbes and cells; and the adherence of the particles onto the surfaces of the microbes and cells of plants. By contrast, normal Mg(OH)₂ with grain sizes of 0.1 to 100 microns generally have surfaces which are dominated by the stable 001 surface, and the concentration of ROS would be small.

The same mechanisms ascribed above to nano-Mg(OH)₂ may apply to other bioactive materials based on metal oxides, such as nano-ZnO and AgO. Their nanograms will also support a range of ROS species that depend on the specific defects at the respective grain boundaries. For example, nano-ZnO is known to produce peroxyl and hydroxyl radicals.

The mechanism for bio-activity of nano-grain particles is substantially different from most other fungicides and bactericides, which use toxic compounds to target pathogenic microbes. Firstly, the mechanism of ROS lies at the core differentiation between aerobic and anaerobic microbes, and genetic evolution of pathogens to limit the impact of the bio-activity is unlikely, so that the bioactivity of nano-MgO for a given pathogen is unlikely to diminish. Secondly, the mechanism is an enhancement of the natural processes whereby plants defend themselves against pathogenic attack. No new chemical species are involved, and the products of the decomposition are essential nutrients or micro-nutrients, and in the case of magnesium, it is an essential nutrient for the production of chlorophyll. Plants absorb magnesium through stomata on the leaves, and the aerobic/anaerobic contests between pathogenic fungi, gram-positive and gram-negative microbes and the plant cells take place both within the soil and on the leaves, for example, as described by Susan S. Hirano and Christen D. Upper, Microbiol. Mol. Biol. Rev. 64, 3624-653 (2000).

This disclosure may relate to a formulation which is broadly a mineral probiotic. A probiotic has been defined in proceeding of the US Trademark and Patent Office, Trademark Trial and Appeals Board, Serial 77758863, (2013) a generic name for a fertilizer using friendly bacteria in the soil producing microbial ecology means to bring back symbiotic relationships to the soil. In this disclosure, the definition of probiotic is extended to include symbiotic relationships on the surface of an organism, such as plant leaves, and the symbiosis is specifically associated with the relationship between the organism and the beneficial gram positive bacteria, which are essential to a healthy environment for growth. To that extent, the nano-Mg(OH)₂ is a probiotic, albeit mineral based. Indeed, when nano-Mg(OH)₂ is applied onto leaves as a folia spray, the impact of magnesium absorption as a fertilizer is noticeable through both the colour from increased chlorophyll, and the increased leaf thickness. Thus at a technical level, for agriculture the properties of nano-Mg(OH)₂ satisfies the requirements of being both a fertiliser and an pathogen inhibitor. The definition can be extended to other ecosystems, such as aquatic ecosystems, where the probiotic effect may include the provision of calcium and magnesium to the host species, which may or may not include human organs.

The means of production of nano-materials use chemical synthesis, and the materials are expensive to produce. Furthermore, the handling of very fine powders is difficult because these powders have a tendency to readily float in air. Most importantly, nano-materials are very difficult to filter from air using conventional air filters. Thus the production processing of these materials requires expensive handling equipment to avoid loss of the materials, and to meet safety, health and environmental regulations. These costs are such that nano-materials have not made a substantial impact in the markets for biocides. Equally important, there are concerns about nano-particles arising from their ability to be absorbed through the skin, and inhaled into the lungs, by virtue of their small size. The application of nano-particles to health care products has been of great community concern. Materials such as magnesium hydroxide are used in such products, but not generally for their bio-activity against fungi, bacteria and viruses. It is desirable to produce a material that exhibits the same biological impact as nano-particles, without any of the concerns of nano-particles. In a nanoparticle, the concentration of the bioactive constituents is an important consideration. In the case of Mg(OH)₂ nano-particles, there is evidence that the bioactive species, such as magnesium peroxide is determined by the concentration of defects at the grain boundaries of the oxide precursors, and the amount of these defects will increase as the nanoparticle size decreases because the defects appear on the unstable crystalline surfaces and the boundaries of surfaces. The concentrations of these species is difficult to measure, especially when recombination of reducing species, such as F-centre electrons, with the oxidising species may occur. The concentration of ROS produced is preferably small, and comparable to those found in a healthy ecosystem when infestation has been overcome. The need for a limit in the ROS production arises because ROS species readily diffuse into cells of the host and can attack the cellular structure. ROS is generated in the normal product of cellular metabolism. Excessive ROS is known to damage DNA, oxidise polyunsaturated fatty acids in lipids, oxidise amino acids in proteins, and oxidatively inactivate specific enzymes by oxidation of co-factors, for example as described by L. Packer and E. Cadensas "Understanding the process of aging: the roles of mitochondria, free radicals and antioxidants", New York, Marcel-Dekker, 1999. While the aerobic cells of the host have mechanisms of quenching such radical species in order to live in an oxygenated atmosphere, their ability to withstand very high concentrations of ROS is limited. For example, hydrogen peroxide and magnesium peroxide are both commercially available materials, and can be used to sterilise environments because they can kill all the microbes, both aerobic and anaerobic when applied, to humans, plants and animals they may induce a broad response of symptoms the alleviation of which is only accommodated only by extensive washing of any exposed surfaces.

It would seem that the non-toxic impact of nano-Mg(OH)₂ is achieved by maintaining the ROS precursors, believed to by magnesium peroxide, at a level that can kill or retard the growth of pathological organism in the ecosystem, but is below a level which causes a significant amount of ROS to diffuse into the cells of the host. The role is to constrain the ROS concentration at a level that is comparable to that of a healthy aerobic ecosystem in which the host's protective structure and responses are adapted. The use high peroxide particles, such as those that are commercially available as magnesium or calcium peroxide creates an excessive local concentration of ROS, and sterilises the surface and induces a response from the host. Dilution of commercial sterilisers such as peroxide particles is not effective because the local concentration is orders of magnitude too high. On the other hand, the observations of a weak transient bio-response when hydroxides formed from magnesite or dolomite suggests that the naturally occurring ROS precursors in such substances is not sufficiently high. This invention seeks to overcome the limitation on bioactivity of using either magnesium peroxide (bioactivity too strong) or magnesium oxide (bioactivity too weak), and to avoid the use of nano-Mg(OH)₂ (bioactivity ideal). The prior cited art mentioned above refers to the mechanism of the bioactivity as being a biocide. However, the mode of action in the field is arguably as a protective mode, rather than curative as would occur from traditional biocides, which are targeted on killing the pathogen. The ability of nanoparticles to provide an environment which inhibits either the growth of a pathogen on a surface of a plant of animal, or the transport of the pathogen through the surface, is of importance to this disclosure. Thus the mode of action in this disclosure may be a pathogen inhibitor, and the positive impact of the nanomaterial on the ecosystem of the surface is that of a protective mineral-based material, as a mineral based probiotic. Within plants, animals and humans, the Mg(OH)₂ may be readily dissolved by the acids within the organism, such that systemic effects typically do not occur.

It may be important to note that the pH of Mg(OH)₂ is about 10.4 and that of hydrated lime, Ca(OH)₂ is about 12.0. Hydrated lime, as in whitewash, cannot be used as a pathogen inhibitor because its extreme pH is toxic to an ecosystem, killing both the aerobic and anaerobic micro-organisms, and a response from the host to minimize phytotoxic effects, such as 'leaf burning' in agriculture when applied to leaves, and pain to animals and humans when applied to tissues such a skin.

The fact that nano-Mg(OH) does not induce such a phytotoxic response, at the dose rates required for a biocide of pathogenic anaerobic micro-organisms, suggests that the local pH of 10.4 is tolerable to a number of tissues. This means that nano-Mg(OH)₂ will be readily consumed by reaction with the acids that are characteristic of the healthy ecosystems on surfaces, where the pH is below about 7.0. The small particles in nano-Mg(OH)₂ act as a biocide for a brief period of time because of acid neutralisation, and cannot maintain an aerobic ecosystem over an extended period of time at the dose rates compatible with biocidal action against a pathogen present when applied.

There is a need for a product that has the same desirable intrinsic biological activity of nano-materials using an industrial scale process that can produce significant volumes of product at a reasonable cost, but that also avoids the cost and material-handling issues of nano-materials, and their potential for absorption and inhalation, and which provides a product that, when applied to various ecosystems of importance to agriculture, aquaculture and health care, provide a long lived and healthy ecosystem that is able to resist disease.

There is no universally accepted definition of a nanomaterial, so for the purpose of this invention, a 'nanomaterial' is defined as a natural, incidental or manufactured material containing particles, in an unbound state or as an aggregate or as an agglomerate and where, for 50% or more of the particles in the number size distribution, one or more external dimensions is in the size range 1 nm-100 nm; a 'particle' means a minute piece of matter with defined physical boundaries; an 'agglomerate' means a collection of weakly bound particles or aggregates where the resulting external surface area is similar to the sum of the surface areas of the individual components; and an 'aggregate' means a particle comprising of strongly bound or fused particles.

The measure of whether the particles in the slurry are not nanoparticles is that the specific surface area by volume of the dried particles, as a powder, by volume is less than 60 m²/cm³. The specific surface area by weight is measured by the BET surface area in m²/g and the bulk density, in g/cm³ of the dried powder, suitably disaggregated, is measured using standard powder bulk density methods. The product of the BET surface area, and the bulk density is the specific surface area by volume. This definition and methodology is that recommended by the European Union in its COMMISSION RECOMMENDATION of 18 October 2011 on the definition of nanomaterial (2011/696/EU). Other definitions are generally less demanding, and generally do not adequately deal with the practical issues of agglomeration and/or aggregation.

Any discussion of prior art throughout the specification should in no way be considered as an admission that such prior art is widely known or forms part of the common general knowledge in the field.

### SUMMARY

### PROBLEMS TO BE SOLVED

A problem to be solved is the formulation of a product that can act as a pathogen inhibitor, and which is not a nanomaterial, and thereby avoids the cost and materials handling issues and possible health risks of nanoparticles, but which provides the equivalent biological activity of a nanomaterial, specifically when applied to a surface as a pathogen inhibitor with the objective of reducing the incidence of infection, rather than a curative like a systemic fungicide. The ecosystems of interest are plant roots and leaves for agriculture; water, pond bottoms and the skin of fish for aquaculture, and human tissue for health and medical products.

It may be an object or aim of the present invention to at least address or ameliorate at least one or more problems associated with the prior art or background formulations.

### MEANS FOR SOLVING THE PROBLEM

A first aspect of the present invention provides a formulation for producing a pathogen inhibitor concentrate which may be adapted for use in agricultural applications, wherein the formulation includes a mixture of solids and water in a slurry, in which the particles contain preferably a uniform distribution of magnesium hydroxide and magnesium peroxide with a relative molar ratio that may be formulated to provide sufficient alkali and reactive oxygen species to maintain a healthy leaf ecosystem, and to provide magnesium to the plant as a fertiliser, when diluted and sprayed onto the leaves of a plant as a folia spray.

A second aspect of the present invention provides a further formulation for producing a pathogen inhibitor concentrate which may be adapted for use in aquaculture applications wherein the formulation includes mixture of solids and water in a slurry, in which the particles may preferably contain a generally uniform distribution of magnesium hydroxide, calcium carbonate and magnesium peroxide with a relative molar ratio that may be formulated to provide sufficient alkali and reactive oxygen species to maintain. a healthy water ecosystem, and to provide magnesium and calcium to fish as a food, when diluted and dosed or introduced into the water.

A third aspect of the present invention provides a further formulation for producing a pathogen inhibitor which may be adapted for use in medical and heath applications, wherein the formulation containing the bioactive particles may be used as either as a paste which may be applied directly to tissue, such as skin, for example, or used as a setting compound, such as a bandage or a gauze. The formulation may comprise bioactive particles which preferably contain a generally uniform distribution of magnesium hydroxide (Mg(OH)₂) and magnesium peroxide (MgO₂) with a relative molar ratio that may be formulated to provide sufficient alkali and reactive oxygen species to maintain a protective barrier against pathogen migration. Examples may include ointments in which the formulation when applied to the skin suppress the growth of skin diseases, such a tinea and dandruff, or when applied to a gauze, it sets to form a barrier to air borne pathogens such as influenza, bird flu, and the like.

A particular aspect of the present invention provides a pathogen inhibitor concentrate for use in an ecosystem, the concentrate comprising particles having a uniform distribution of magnesium hydroxide and magnesium peroxide with a relative molar ratio to control the bioactivity of the concentrate by providing sufficient alkali and reactive oxygen species to meet the requirements of the ecosystem, wherein the requirements of the ecosystem are defined as maintaining an aerobic system, killing pathogenic, anaerobic microbes or producing a sterile ecosystem, and the requirements of the ecosystem are determined based on the redox potential and pH of the ecosystem, wherein at least one of the magnesium hydroxide and the magnesium peroxide make up at least 30% by weight of the concentrate.

In an embodiment, the generation rate of the reactive oxygen species can be controlled by particle size distribution and particle surface area.

In an embodiment, the particles are of a particle size distribution in the range of about 0.3-100 microns. More preferably, the particle size distribution includes a mean particle size of between 10 and 20 microns. In an example, the content of solids in the formulation is at least 35% by weight, and preferably 60% by weight.

In an embodiment, the concentrate is diluted to 2% by weight with a fluid for use as an agricultural spray to be sprayed onto the leaves of a plant as a folia spray. In an example, the magnesium hydroxide content is at least 50% by weight.

The combined magnesium hydroxide and calcium carbonate content may be at least 80%, and the calcium to magnesium ratio on a molar basis may be at least 35%.

In an embodiment, the magnesium peroxide is formed by adding at least one precursor to generate the magnesium peroxide. The precursor may be a reactive precursor comprising hydrogen peroxide.

Preferably, the magnesium peroxide to magnesium hydroxide content on a molar basis may be determined by the requirement of on a molar basis to maintain the health of the respective ecosystem, including the presence or potential presence of pathogenic microbes that would otherwise induce infection and possible necrosis of the host unless the formulation was applied. The health of the ecosystem can generally be measured by the Redox Potential and the pH of the ecosystem.

Preferably, the dose rate of the concentrate is not less than 3 kg/Ha per application (that is, at least 3 kg/Ha per application), and the time between applications determined solely by the loss of coverage of the powder on the leaf (such as with at least 10% coverage). Preferably, the process of manufacture of the slurry, without the need for specific addition of compounds that produce magnesium peroxide. This process is described in PCT/AU2014/001115 (published as WO 2015/100468), which is incorporated herein by reference. If the preferred magnesium peroxide does not meet the predetermined requirements, the additional magnesium peroxide is added, or formed by the addition of precursors such as hydrogen peroxide, as described in this disclosure.

A further aspect of the present invention provides a further formulation for producing a biocide concentrate which may be adapted for use in aquacultural applications wherein the formulation includes mixture of solids and water in a slurry, in which the particles contain preferably a uniform distribution of magnesium hydroxide, calcium carbonate and magnesium peroxide with a relative molar ratio that may be formulated to provide sufficient alkali and reactive oxygen species to maintain a healthy water ecosystem, and to provide magnesium and calcium to the fish as a food, when diluted and dosed into the water.

In an embodiment, the particles are of a particle size distribution in the range of about 0.3 -100 microns. More preferably, the particle size distribution includes a mean particle size of between 10 and 20 microns. In an embodiment, the content of solids in the formulation is at least 35% by weight, and preferably 60% by weight. The preferred concentrate may be diluted to 2% for spraying, or may be dosed into water systems to achieve such a concentration, or may be applied as a coating with other additives. Preferably, the magnesium hydroxide content may be at least 80% by weight.

The combined magnesium hydroxide and calcium carbonate content may be at least 80%, and the calcium to magnesium ratio on a molar basis may be at least 35%. In an embodiment, the magnesium peroxide to magnesium hydroxide content on a molar basis may be determined by the requirement of on a molar basis to maintain the health of the respective ecosystem, including the presence or potential presence of pathogenic microbes that would otherwise induce necrosis of the host unless the formulation was applied. In an embodiment, the dose rate of the concentrate is not less than 60 kg/ML per application, and the time between applications may be determined solely by the pH of the water, which should preferably be maintained between 6.0 and 8.0, and more preferably 7.5.

A further aspect of the present invention provides a formulation of a slurry of particles, which are not nano-particles, in which the bioactivity of the formulation may be similar to that of nanoparticles. In this specification, references to the term "pathogen inhibitor" may be any material adapted to beneficially promote or enhance the microbial balance within the treated area, location or place to inhibit infection of the host. More particularly, the term "pathogen inhibitor" means an inorganic alkali salt compound which may inhibit, limit growth or kill pathogens including bacteria, viruses, or fungi.

### DETAILED DESCRIPTION

The invention will be better understood and readily apparent to a person skill in the art from the following description of embodiments of the invention, provided by reference to the non-limiting examples. The invention is related to a slurry of solid in water, which is a concentrate which is diluted for spraying or dosing. The solids comprise a hydrated metal oxide and one or more precursors for the generation of ROS.

The preferred product is a stable, 60% solids slurry as a concentrate, with a particle size distribution preferably between 0.3 and 100 microns in which the ROS precursors are uniformly distributed in each particle. The viscosity of the concentrate should be less than 250 cP, and preferably less than 100 cP to allow for use in dosing systems, and for storage and transport. The formation of a gel, and syneresis, should be minimised. The resistance of the gel that does form should be low, so that the product can be readily fluidised by stirring. The production of magnesium hydroxide slurry with these properties is described by Sceats and Vincent in AU 2013904096 (incorporated herein by reference). An object of this invention is to optimise the bioactivity of such slurries.

The primary basis for the examples are that the ROS precursors in the particles can be intrinsic precursors formed during the preparation of the oxide and the hydration of the oxide to form the slurry, and, in addition, precursors formed by a synthetic precursors using a reagent added during this slurry preparation process. It will be apparent to a person skilled in the art that the control of the ROS precursor concentration in the particles is desirable, given the sensitivity of the ecosystems described above to ROS. Such a control during manufacturing may produce a slurry that has a specified redox potential to achieve a mild bioactivity, and which the ROS concentration is used to sustain an existing aerobic environment, or a slurry with an intermediate bioactivity in which the ROS is sufficiently high that pathogenic, anaerobic microbes are killed, but aerobic bacteria that provide positive synergy to the ecosystem are not adversely impacted, or a slurry which has a very high ROS concentration that kills all the microbes in the ecosystem, and which produces a sterile surface. Users of these products can determine the impact they wish to have, ranging from a preventative measure which sustains an ecosystem, to a high impact measure, which combats pathogenic colonies that have taken control of the ecosystems, and which may already have induced infection and possible necrosis to the host organism. In the case of magnesium, the dosage of magnesium for fertilisation can therefore be independently controlled. This flexibility is an important feature of the invention disclosed herein.

Another basis for the examples is the controlled release of the ROS into the ecosystem. Controlled release is a known art in pharmaceuticals in which an active compound, generally uniformly distributed into a particle, is released as the particle is slowly dissolved. The particle size distribution can be used to produce the desired response. Dissolution occurs from the external surface at a constant rate (in m/s), for particles having the same surface area. Thus large particles can provide a source of ROS over an extended period of time, as all the particles gradually dissolve. Magnesium hydroxide is relatively insoluble in water, and is gradually dissolved because it is a source of alkali, and is therefore consumed at a rate that depends on the difference of the pH of the ecosystem compared to the intrinsic pH of magnesium hydroxide, of 10.4. The product user can specify the duration required for the generation of ROS through specification of the particle size distribution and the particle surface area. This capability does not exist for nano-particles .

Consider firstly the production of ROS from intrinsic precursors, for the case of a magnesium hydroxide slurry. The prior art shows that a slurry produced from conventional caustic magnesia produces a transient biocidal impact, and this arises from the low density of ROS precursors because of the low density of defects on the large stable surfaces of the magnesia. The primary indicator of the density of defects is the specific surface area by volume of the particles, SSAv. Thus an SSAv of 400 m²/cm³ in the magnesia will have a higher precursor density than magnesia with an SSAv of 10 m²/cm³. The SSAv of magnesia can be controlled by sintering of the magnesia, which causes the micropores of the particles to collapse to form mesopores as the surface energy is reduced, and it is the reduction of the surface energy that eliminates the crystal defects that generate ROS during hydration. The highest ROS will be generated from the highest SSA materials, and there are a number of production processes that can be used to produce high surface area magnesia. One such process is described by Sceats and Horley, in WO 2007/112496 (incorporated herein by reference), using flash calcination of magnesite in an indirectly heated reactor in which the external heating gas flows in a counterflow to the calcining particles. Sintering of those particles at a high temperature, in steam or CO₂, can be used to produce the desired SSAv. The particle size distribution can be controlled during the grinding process, either pre- or post-calcination, or as part of a hydration process during wet grinding of magnesia.

Consider secondly the synthesis of the ROS precursors. It is desirable that the reaction takes place homogeneously within the particle in such a way that the active species is formed uniformly with the magnesia particle. In the case where magnesium peroxide is the active compound, hydrogen peroxide can be used to react with magnesia to form magnesium peroxide in an exothermic reaction. This process is a known art, and produces, if allowed to go to completion, particles which are about 30% magnesium peroxide by weight. The magnesium peroxide is stable below 100°C, above which it begins to decompose to give oxygen, and the reaction is generally carried out at 50°C. The synthesis route is not prescribed in this invention. Production may take place as a gas phase reaction, or in the slurry. For high surface area magnesia, the preferred process is a gas phase process on the very high porosity magnesia particles at a temperature and pressure such that the homogenous material is produced. Alternatively, in a wet grinding process using large magnesium oxide granules, the hydrogen peroxide may be added to the water and reacts with the particles as they are ground and hydrated, or is added in a subsequent synthetic process. Generally, the hydrogen peroxide is dosed into the system to give the desired concentration of ROS precursors in the particle, including the intrinsic ROS .

Another approach to control the precursors is to heat a powder containing excess magnesium peroxide to about 150°C, for a controlled time, to decompose the peroxide sufficiently to achieve the desired activity. Alternative, a slurry of such material can be heated to about 70°C for a period of time. In both cases, the excess peroxide forms oxygen gas. Heating can be used be adjust the activity of any powder or slurry to meet the requirements of the application.

For applications in aquaculture, the size distribution of the particles is important. Small particles, typically less than 5 microns, are buoyed by Brownian motion, move with currents, and drift slowly downwards under the force of gravity. Larger particles fall quickly to the base of the pen. This the residence time of a particle in each of the ecosystems depends on the particle size. The particle size distribution, such a fine or coarse grade, can be optimised for delivery of ROS and alkali to each system as required. In addition, the growth of fish is determined by the calcium content of the ingested food, and the particles can be processed from dolomite as a feedstock to produce a particle in which the degree of calcination of the magnesium site and the calcium site is controlled to selectively remove CO₂ from the magnesium oxide site to form a material semidolime MgO.CaCO₃. When processed to a slurry with the desired ROS levels, the product has both magnesium and calcium as nutrients for the growth of the fish. The stock in the pond benefits from the nutrients, as well as from living in the water and pond base ecosystems that are healthy and aerobic.

The pathogen inhibitor activity of the slurry has been established using in vitro measurements and in preliminary crop trials. For in vitro studies, the slurry is diluted to 2% by the addition of water, and is sprayed into a prepared Petri dish in which a dot of the fungus, bacteria, or virus strain under test has been incubated and grown over 24 hours. The rate of growth of the radius is measured over a period, and the biocidal impact is measured by the extent that the ring growth rate has been suppressed. Studies were completed on a number of fungi, and a broad spectrum antifungal impact was observed, and is comparable to commercial fungicides.

For preliminary crop trials, a number of crops such as grapes, avocados and bananas exhibiting fungal outbreaks were sprayed with the diluted slurry, and the biocidal impact measured by the healthiness of the crop, especially with regard to the presence of fungi, compared to a field that was not sprayed. On inspection, after 7 days, the fungi were not observable on the sprayed area. It was noted that the powder had a strong adherence to leaves, and that the leaf appearance had improved indicating that the magnesium was being adsorbed into the plant and promoting greater photosynthesis. Such leaf characteristics include the colour and leaf thickness.

In trials of insecticide response, a sample of insect ridden wheat was dusted with magnesium oxide powder. After several days, the insect count had decreased considerably, and with a response that was similar to dehydrated diatomaceous earth.

The slurry described in this invention is not generally deployed as a pathogen inhibitor at 60% solids. It is a concentrate that is used to make pathogen inhibitors for different applications. The means of application of pathogen inhibitors in agriculture is preferably through a sprinkling system to avoid losses to the crop from wind. A common means is to use a slurry of the materials, which is diluted by the spray water to about 2%. This foliar spray approach has wide industry acceptance. In that case, a material based on magnesium hydroxide has an added benefit of providing a source of magnesium, which is an essential nutrient for photosynthesis. A spray should preferably have particles that are less than 100 microns, and preferably 25 microns, diameter to avoid blockage of the nozzle. The use of a spray may also be applicable for medical applications. However, in that area, there is also an application for the incorporation of the material in a mask to reduce infection from airborne microbes, or a wipe to remove microbes from surfaces.

In another application, the slurry should be mixed with existing biocides as adjuvents. This includes conventional water soluble biocides, typically molecular, which adsorb onto the particle to deliver a desired biocide activity. The formation of emulsions with oils that contain oil soluble adjuvents is another such application.

In a further embodiment, the aforementioned slurry may be mixed with other compounds to form a topical cream for the treatment of wound dressings. Preferably, the water component of the abovementioned slurry may further incorporate a standard topical cream additives including sorbolene.

In a further embodiment, the slurry may be applied to a wound dressing wherein a medical bandage is soaked for at least 10 minutes in a solution or formulation comprising the aforementioned slurry. The bandage then preferably takes on some of the pathogen inhibitor qualities of the slurry.

In this specification, the word "comprising" is to be understood in its inclusive sense, and thus not limited to the sense of "consisting only of'. A corresponding meaning is to be attributed to the corresponding words "comprise", "comprised" and "comprises" where they appear.

While particular embodiments of this invention have been described, it will be evident to those skilled in the art that the present invention may be embodied in other specific forms without departing from the essential characteristics thereof. The present embodiments and examples are therefore to be considered in all respects as illustrative and not restrictive, with all changes which come within the meaning and range of equivalency therefore intended to be embraced therein.

Although the invention has been described with reference to specific examples, it will be appreciated by those skilled in the art that the invention may be embodied in many other forms, within the scope of the following claims.

The present invention and the described preferred embodiments specifically include at least one feature that is industrial applicable.

## Claims

1. A pathogen inhibitor concentrate for use in an ecosystem, the concentrate comprising particles having a uniform distribution of magnesium hydroxide and magnesium peroxide with a relative molar ratio to control the bioactivity of the concentrate by providing sufficient alkali and reactive oxygen species to meet the requirements of the ecosystem, wherein the requirements of the ecosystem are defined as maintaining an aerobic system, killing pathogenic, anaerobic microbes or producing a sterile ecosystem, and the requirements of the ecosystem are determined based on the redox potential and pH of the ecosystem, wherein at least one of the magnesium hydroxide and the magnesium peroxide make up at least 30% by weight of the concentrate.

2. The pathogen inhibitor concentrate of claim 1 wherein the generation rate of the reactive oxygen species can be controlled by particle size distribution and particle surface area.

3. The pathogen inhibitor concentrate wherein the particles are of a particle size distribution in the range of about 0.3-100 microns.

4. The pathogen inhibitor concentrate of claim 3, wherein the particle size distribution includes a mean particle size of between 10 and 20 microns.

5. The pathogen inhibitor concentrate of claim 4, wherein the content of solids is at least 35% by weight, and preferably 60% by weight.

6. The pathogen inhibitor concentrate of claim 1 wherein the concentrate is diluted to 2% by weight with a fluid for use as an agricultural spray to be sprayed onto the leaves of a plant as a folia spray.

7. The pathogen inhibitor concentrate of claim 6, wherein the magnesium hydroxide content is at least 50% by weight.

8. The pathogen inhibitor concentrate of claim 7, wherein the combined magnesium hydroxide and calcium carbonate content is at least 80% by weight, and the calcium to magnesium ratio on a molar basis is at least 35%.

9. The pathogen inhibitor concentrate of claim 1, wherein the magnesium peroxide is formed by adding at least one precursor to generate the magnesium peroxide.

10. The pathogen inhibitor concentrate of claim 9, wherein the precursor is a reactive precursor comprising hydrogen peroxide.

11. The pathogen inhibitor concentrate of claim 1, wherein the magnesium peroxide to magnesium hydroxide content on a molar basis is determined by the requirement of on a molar basis to maintain the health of the respective ecosystem, including the presence or potential presence of pathogenic microbes that would otherwise induce necrosis of the host unless the formulation was applied.

12. The pathogen inhibitor concentrate of claim 6, wherein the dose rate of the concentrate is at least 3 kg/Ha per application, and the time between applications is determined solely by the loss of coverage of the powder on the leaf.

13. The pathogen inhibitor concentrate of claim 6, wherein the fluid comprises water.

14. The pathogen inhibitor concentrate of claim 1 for use as a formulation for a topical ointment and a bandage formulation.
